(19)

Europäisches Patentamt

European Patent Office

Office européen des brevets

(11) **EP 4 744 685 A1**

(12) **EUROPEAN PATENT APPLICATION**

(43) Date of publication:
    **20.05.2026  Bulletin 2026/21**

(21) Application number: **24213143.1**

(22) Date of filing: **15.11.2024**

(51) International Patent Classification (IPC):
    **A61L 15/24** (2006.01)      **A61L 15/46** (2006.01)
    **A61L 15/60** (2006.01)

(52) Cooperative Patent Classification (CPC):
    (C-Sets available)
    **A61L 15/60; A61L 15/24; A61L 15/46;**
    A61L 2300/208; A61L 2300/404; A61L 2300/41
                                                    (Cont.)

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **GE KH MA MD TN**

(71) Applicant: **Paul Hartmann AG**
    **89522 Heidenheim (DE)**

(72) Inventor: **KETTEL, Markus**
    **89520 Heidenheim (DE)**

(74) Representative: **Paul Hartmann AG**
    **Patents & Licensing**
    **Paul-Hartmann-Straße 12**
    **89522 Heidenheim (DE)**

(54) **PARTICLES COMPRISING A SUPERABSORBENT POLYMER AND A QUATERNARY AMMONIUM COMPOUND**

(57)     The present invention relates to particles comprising a superabsorbent polymer (SAP) and a quaternary ammonium compound (Quat). The superabsorbent polymer is a crosslinked polyacrylate. The quaternary ammonium compound is antimicrobial and/or anti-inflammatory. Moreover, the quaternary ammonium compound is non-covalently linked to the superabsorbent polymer. The particles can thus be advantageously highly absorbent, antimicrobial, anti-inflammatory, easy to produce and inexpensive. Furthermore, the present invention relates to a process for producing these particles and a wound dressing comprising these particles.

Figure 24

**(Cont. next page)**

(52) Cooperative Patent Classification (CPC): (Cont.)

C-Sets
**A61L 15/24, C08L 33/02**

**Description**

The present invention relates to particles comprising a superabsorbent polymer and a quaternary ammonium compound. Furthermore, the present invention relates to a process for producing these particles and a wound dressing comprising these particles.

Superabsorbent polymers (SAP) in particle form are known in the prior art. They can bind large amounts of fluid and are used in wound dressings, among other things. Accordingly, SAP-containing wound dressings have a high absorption capacity for wound fluid and can be used advantageously for the treatment of heavily exuding wounds. Such wounds can also be infected or inflamed. There is therefore a need to provide SAP-containing wound dressings with antimicrobial and anti-inflammatory properties.

The task of the present invention was to provide novel SAP particles for medical purposes, in particular for wound treatment. The SAP particles should be able to have an antimicrobial and/or anti-inflammatory effect. In addition, the SAP particles should be easy to produce and inexpensive. The aforementioned tasks are solved according to the invention with the particles according to claim 1.

According to the invention, the particles comprise a superabsorbent polymer (SAP) and a quaternary ammonium compound (Quat). The superabsorbent polymer is a crosslinked polyacrylate. The quaternary ammonium compound is antimicrobial and/or anti-inflammatory. Moreover, the quaternary ammonium compound is non-covalently linked, in particular solely non-covalently linked, to the superabsorbent polymer. Therefore, usually no covalent bonds between the quaternary ammonium compound and the SAP are present in the particles according to the invention. The particles can thus be advantageously highly absorbent, antimicrobial, anti-inflammatory, easy to produce and inexpensive. Further advantages result from the following preferred embodiments of the invention.

Typically, the particles are substantially anhydrous or dry. Nevertheless, the particles according to the invention may still contain a small amount of water, for example up to about 6 % by weight water, up to about 5 % by weight water or up to about 4 % by weight water. This moisture content of the particles may be determined according to NWSP 230. Furthermore, the particles may have a particle size of 100 to 1,000 $\mu$m, wherein the specified range can refer to the average particle size. In particular, the particles may have a particle size distribution of 150 to 850 $\mu$m, which may be determined according to NWSP 220. In a preferred embodiment of the invention, the particles consist essentially of the superabsorbent polymer and the quaternary ammonium compound.

The superabsorbent polymer is advantageously a crosslinked sodium polyacrylate. The superabsorbent polymer can also be a copolymer of acrylic acid or, in particular, sodium acrylate and acrylamide. Suitable SAP particles made from such a crosslinked sodium polyacrylate are offered by Evonik, for example, under the name FAVOR®-PAC 300 (CAS-No. 9003-04-7).

Preferably, the quaternary ammonium compound is antimicrobial and anti-inflammatory at the same time. The anti-inflammatory efficacy of the quaternary ammonium compound may be due to the quaternary ammonium compound being able to bind a bacterial endotoxin and/or a matrix metalloproteinase (MMP). Accordingly, the quaternary ammonium compound can advantageously bind a bacterial endotoxin and/or a matrix metalloproteinase. In the present case, particular emphasis is placed on lipopolysaccharides (LPS) and lipoteichoic acids (LTA) as endotoxins and MMP-2 and MMP-9 as matrix metalloproteinases. Therefore, the quaternary ammonium compound can advantageously bind LPS, LTA, MMP-2 and/or MMP-9.

The quaternary ammonium compound may be selected from the group consisting of benzalkonium chloride (CAS-No. 63449-41-2), benzethonium chloride (CAS-No. 121-54-0), cetalkonium chloride (CAS-No. 122-18-9), tetradecyltrimethylammonium bromide (CAS-No. 1119-97-7) and polyquaternium 10 (CAS-No. 68610-92-4). These compounds can provide the SAP particles with antimicrobial and/or anti-inflammatory properties, as shown here. Benzalkonium chloride and tetradecyltrimethylammonium bromide are particularly preferred.

Usually, the quaternary ammonium compound is linked to the superabsorbent polymer via ionic interactions. In particular, a positively charged functional group of the quaternary ammonium compound, in particular a quaternary nitrogen group, is linked to a negatively charged functional group of the superabsorbent polymer, in particular a carboxyl group, via ionic interactions. The connection of the quaternary ammonium compound with the superabsorbent polymer via ionic interactions or ionic forces of attraction is easy to implement and can still be durable and stable.

The particles may contain 60 to 97.5 % by weight SAP, based on the total weight of the particles. Preferably, the particles may contain 60 to 90 % by weight SAP, based on the total weight of the particles. More preferably, the particles may contain 60 to 80 % by weight SAP, based on the total weight of the particles. In particular, the particles may contain 60 to 70 % by weight SAP, based on the total weight of the particles.

The particles may contain 2.5 to 40 % by weight of the quaternary ammonium compound, based on the total weight of the particles. Preferably, the particles may contain 10 to 40 % by weight of the quaternary ammonium compound, based on the total weight of the particles. More preferably, the particles may contain 20 to 40 % by weight of the quaternary ammonium compound, based on the total weight of the particles. In particular, the particles may contain 30 to 40 % by weight of the quaternary ammonium compound, based on the total weight of the particles.

Accordingly, the particles according to the invention may comprise or essentially consist of

- 60 to 97.5 % by weight SAP, based on the total weight of the particles,
- 2.5 to 40 % by weight of the quaternary ammonium compound, based on the total weight of the particles, and
- optionally up to 6 % by weight water, based on the total weight of the particles.

Preferably, the particles according to the invention may comprise or essentially consist of

- 60 to 90 % by weight SAP, based on the total weight of the particles,
- 10 to 40 % by weight of the quaternary ammonium compound, based on the total weight of the particles, and
- optionally up to 6 % by weight water, based on the total weight of the particles.

More preferably, the particles according to the invention may comprise or essentially consist of

- 60 to 80 % by weight SAP, based on the total weight of the particles,
- 20 to 40 % by weight of the quaternary ammonium compound, based on the total weight of the particles, and
- optionally up to 6 % by weight water, based on the total weight of the particles.

In particular, the particles according to the invention may comprise or essentially consist of

- 60 to 70 % by weight SAP, based on the total weight of the particles,
- 30 to 40 % by weight of the quaternary ammonium compound, based on the total weight of the particles, and
- optionally up to 6 % by weight water, based on the total weight of the particles.

In addition, the particles may have an absorption capacity of at least 100 g/g. The upper limit for the absorption capacity of the particles can be 300 g/g, 250 g/g or just 200 g/g. The absorption capacity of the particles can therefore be 100 to 300 g/g, 100 to 250 g/g or 100 to 200 g/g. In this context, the absorption capacity of the particles may be determined according to the test method described in the present document (see point 3 below in the experimental section).

The particles may exhibit a log10 reduction of at least 2, preferably of at least 3 and in particular of at least 4 against S. aureus and/or P. aeruginosa over a test period of 24 hours. The upper limit for the antimicrobial activity of the particles according to the invention can be 6 log levels. Therefore, the particles may exhibit a log10 reduction of 2 to 6, preferably of 3 to 6 and in particular of 4 to 6 against S. aureus and/or P. aeruginosa over a test period of 24 hours. In this context, the log10 reduction may be determined according to the test method described in the present document (see point 5 below in the experimental section).

Moreover, the particles may each comprise a core and a shell, wherein the superabsorbent polymer is contained in the core and the quaternary ammonium compound is contained in the shell. The shell may consist of or may be formed by the quaternary ammonium compound. The core may consist essentially of or may be formed essentially by the superabsorbent polymer. In this embodiment, the superabsorbent polymer is then merely coated with the quaternary ammonium compound. However, the quaternary ammonium compound may be additionally contained in the core as well. In this case, the core may consist essentially of or may be formed essentially by the superabsorbent polymer and the quaternary ammonium compound. Whether the quaternary ammonium compound is also present in the core of the particles can depend in particular on their molecular weight. Thus, quaternary ammonium compounds with a lower molecular weight penetrate more easily into the superabsorbent polymer than quaternary ammonium compounds with a higher molecular weight. In this context, benzalkonium chloride, benzethonium chloride, cetalkonium chloride and tetradecyltrimethylammonium bromide are regarded as quaternary ammonium compounds with a lower molecular weight. In this context, polyquaternium 10 is regarded as a quaternary ammonium compound with a higher molecular weight. Particles in which the quaternary ammonium compound is also present in the core in the form of an impregnation can have a stronger antimicrobial and anti-inflammatory effect and can therefore be particularly advantageous.

The present invention also relates to a process for producing modified particles as previously described, comprising the following steps:

i. Providing superabsorbent polymer particles in the form of a crosslinked polyacrylate.
ii. Providing a quaternary ammonium compound.
iii. Dissolving the quaternary ammonium compound in water to form an aqueous solution of the quaternary ammonium compound.
iv. Dispersing the superabsorbent polymer particles in the solution to form a hydrogel.
v. Drying the hydrogel to obtain a substantially anhydrous or dry modified polymer.
vi. Optionally comminuting and/or sieving the modified polymer to obtain a desired particle size of the modified polymer.

Claim 12 is specifically directed to particles obtained by this process.

The (non-modified) SAP particles provided in step i. may have the same moisture content, particle size and/or particle size distribution as mentioned before with regard to the modified particles according to the invention.

As already mentioned, the particles according to the invention are intended for medical purposes and in particular in the treatment of skin wounds. Accordingly, the present particles are also claimed for use in medicine, in particular in the treatment of wounds, in the sense of a first or second medical indication. The particles can be used particularly advantageously for the treatment of moist, infected and/or inflamed wounds. In particular in the case of inflamed wounds, the particles can be used to bind LPS, LTA, MMP-2 and/or MMP-9 to provide an anti-inflammatory effect.

When the particles according to the invention are used for wound treatment, they are usually integrated into a wound dressing. Therefore, the present invention also relates to a wound dressing comprising the particles according to the invention. The wound dressing may comprise a backing layer and an absorbent layer comprising the particles.

In particular, the absorbent layer may form an absorbent core of the wound dressing, which is surrounded by a sheath. The sheath may advantageously prevent the absorbent core from disintegrating and the modified SAP particles from being released into the wound. A part of the sheath facing away from the wound may form the backing layer of the wound dressing. A part of the sheath facing towards the wound may form the wound contact layer of the wound dressing.

Furthermore, the absorbent core may comprise or consist of cellulose-based fibers, in particular cellulose fibers, and the particles. For instance, the absorbent core may comprise 40 to 70 % by weight cellulose-based fibers and 30 to 60 % by weight of the particles. The absorbent core may also comprise 50 to 60 % by weight cellulose-based fibers and 40 to 50 % by weight of the particles.

In a preferred embodiment of the invention, the aforementioned sheath may be formed from two nonwoven layers which are joined together at their circumference. For example, the two nonwoven layers may be joined together using an adhesive or ultrasonic welding.

In a particularly preferred embodiment of the invention, the wound dressing further comprises particles consisting essentially of crosslinked polyacrylate, in particular crosslinked sodium polyacrylate. Thus, in this particular embodiment, the wound dressing contains the antimicrobial, modified SAP particles according to the invention as well as conventional, non-modified SAP particles. The addition of conventional SAP particles can be advantageous in order to compensate for a possible lower absorption performance of the modified SAP particles. The wound dressing with a mixture of modified and non-modified SAP particles can therefore be antimicrobial, anti-inflammatory and (still) highly absorbent.

Referring to the last embodiment of the invention, the additional non-modified SAP particles are therefore preferably made from the same superabsorbent polymer, which is contained in the modified, antimicrobial and/or anti-inflammatory particles of the invention. The additional non-modified SAP particles may have an absorption capacity of at least 300 g/g. Again, the absorption capacity of the particles may be determined according to the test method described in the present document (see point 3 below in the experimental section). In addition, the non-modified SAP particles may be integrated into the above-mentioned absorbent layer and absorbent core, respectively, as well.

**Examples of modified SAP particles according to the invention and their properties**

To illustrate the present invention, SAP particles modified with various quaternary ammonium compounds were prepared and their properties investigated. However, the examples described below are not intended to be limiting and merely represent embodiments of the present invention.

1. Preparation of modified SAP particles

Modified SAP particles according to the invention were prepared in the following manner. A quaternary ammonium compound was dissolved in demineralized water in a beaker. SAP particles were then added and stirred by hand until the SAP particles had absorbed the entire solution and a hydrogel had formed accordingly. The beaker with the hydrogel was then dried in an oven at 105°C for approximately 12 hours. Finally, if necessary, the dried residue was crushed in a mortar to obtain the modified SAP particles in the desired size.

This process was used to produce modified SAP particles containing 10, 20, 30 or 40 % by weight of benzalkonium chloride (BAC), benzethonium chloride (BEC), cetalkonium chloride (CAC), tetradecyltrimethylammonium bromide (TTABR) or polyquaternium 10 (PQ10). These substances were all purchased from Sigma-Aldrich (Taufkirchen, Germany). The SAP particles used were a cross-linked sodium polyacrylate with the designation "FAVOR®-PAC 300" from Evonik (CAS-No. 9003-04-7).

**Table 1** below contains the quantities of the starting materials used to produce the modified SAP particles. The amount of water used was also influenced by the solubility of the respective quaternary ammonium compound. For example, to produce the modified SAP particles with 90 % SAP and 10 % TTABR (sample 13), 3.34 g of the corresponding quaternary ammonium compound was dissolved in 94.55 g of demineralized water and 30.32 g of SAP was added.

**Table 1:** Quantities of starting materials for the modified SAP particles

| sample No. | weight Quat [g] | weight $H_2O$ [g] | weight SAP [g] |
|---|---|---|---|
| 1 - BAC 10 % | 3.53 | 500.27 | 31.77 |
| 2 - BAC 20 % | 7.89 | 500.06 | 31.56 |
| 3 - BAC 30 % | 13.60 | 500.77 | 31.73 |
| 4 - BAC 40 % | 21.07 | 500.33 | 31.60 |
| 5 - BEC 10 % | 3.34 | 196.10 | 30.10 |
| 6 - BEC 20 % | 7.52 | 225.12 | 30.10 |
| 7 - BEC 30 % | 12.89 | 319.27 | 30.05 |
| 8 - BEC 40 % | 20.08 | 515.30 | 30.09 |
| 9 - CAC 10 % | 2.22 | 450.00 | 20.02 |
| 10 - CAC 20 % | 2.50 | 600.03 | 10.05 |
| 11 - CAC 30 % | 2.58 | 600.05 | 6.10 |
| 12 - CAC 40 % | 2.80 | 836.64 | 4.20 |
| 13 - TTABR 10 % | 3.34 | 94.55 | 30.32 |
| 14 - TTABR 20 % | 7.50 | 90.35 | 30.05 |
| 15 - TTABR 30 % | 12.90 | 95.41 | 30.12 |
| 16 - TTABR 40 % | 20.04 | 419.47 | 30.14 |
| 17- PQ10 10 % | 3.34 | 50.25 | 29.98 |
| 18 - PQ10 20 % | 7.50 | 172.00 | 30.02 |
| 19- PQ10 30 % | 8.61 | 504.63 | 20.01 |
| 20 - PQ10 40 % | 13.36 | 502.12 | 20.03 |

2. Fourier transform infrared spectroscopy

The prepared samples 1 to 20 and, as references, the starting materials were analyzed using Fourier transform infrared spectroscopy (FTIR spectroscopy). The spectrometer used for this was the Tensor 27 from Bruker.

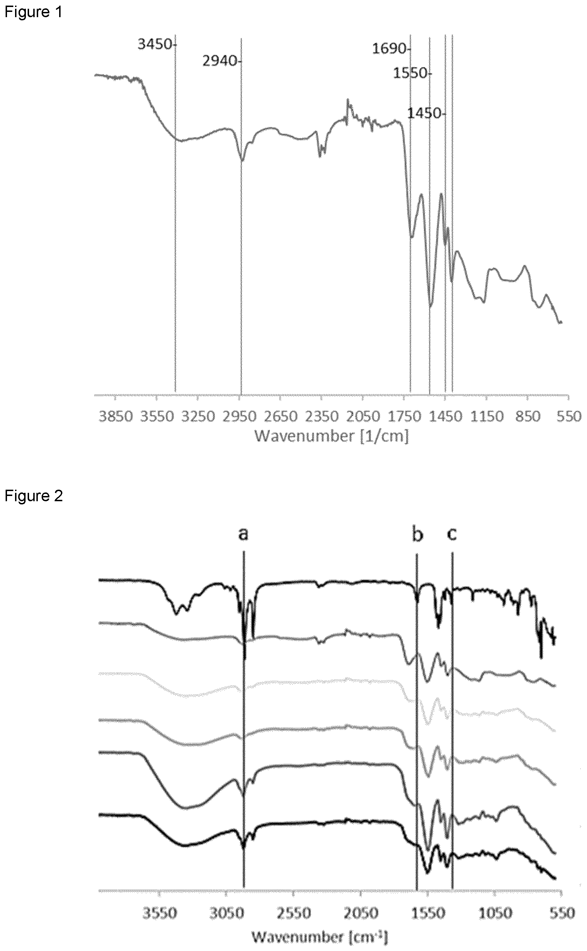

**Figure 1** shows the FTIR spectrum of the used unmodified SAP. Characteristic bands appear at 3450 cm$^{-1}$ (free hydroxyl groups of the water molecules and hydrogen bonds), 2940 cm$^{-1}$ (C-H groups) and 1550 cm$^{-1}$ as well as 1400 cm$^{-1}$ (carboxyl groups).

**Figure 2** shows FTIR spectra of samples 1 to 4 modified with BAC and the FTIR spectra of BAC and unmodified SAP for reference. The spectra are shown as follows (from top to bottom): BAC, SAP, sample 1, sample 2, sample 3, sample 4. In the pure BAC spectra as well as the modified BAC-SAP particles spectra, a peak is observed at 2956 cm$^{-1}$ (a). As the concentration of BAC in the SAP particles increases, the peak becomes more pronounced. This peak is attributed to the alkane C-H stretch. A similar pattern is observed in the peak at 1620 cm$^{-1}$ (b), which is more pronounced in the pure BAC spectra and in 40 % BAC-SAP particles, indicative of the aromatic C=C bond. At 1377 cm$^{-1}$ (c), the observable peak is attributed to tertiary amines.

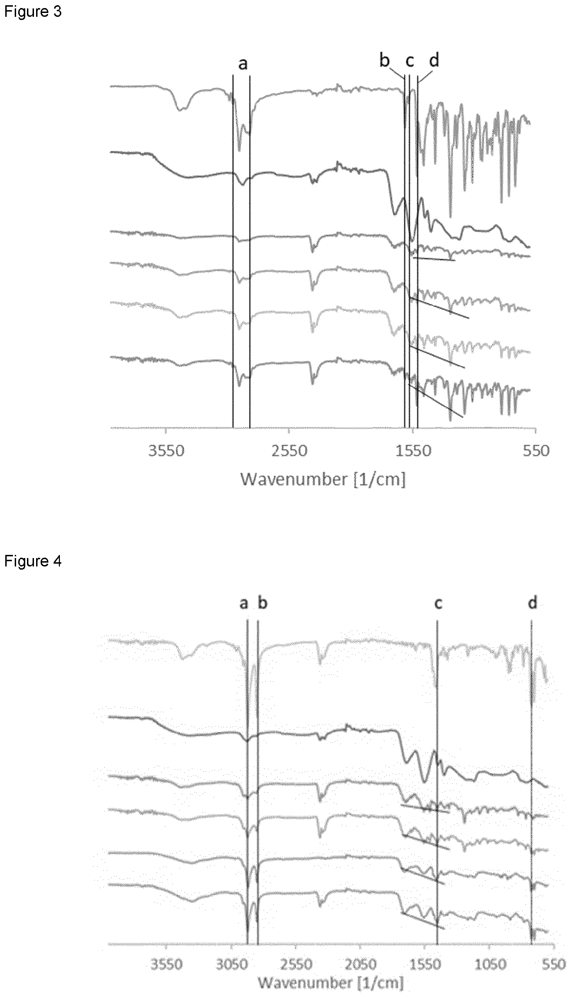

**Figure 3** shows FTIR spectra of samples 5 to 8 modified with BEC and the FTIR spectra of BEC and unmodified SAP for reference. The spectra are shown as follows (from top to bottom): BEC, SAP, sample 5, sample 6, sample 7, sample 8. Characteristic for BEC are the band a in the range from 2953 to 2835 cm$^{-1}$ (C-H groups of the benzene rings as well as aliphatic C-H groups) and in particular the bands b, c and d at 1611, 1579 and 1511 cm$^{-1}$, respectively. The latter three bands are due to the benzene rings of BEC.

**Figure 4** shows FTIR spectra of samples 9 to 12 modified with CAC and, for comparison, the spectra of CAC and SAP alone. The spectra are shown as follows (from top to bottom): CAC, SAP, sample 9, sample 10, sample 11, sample 12. Characteristic for CAC are the bands a and b at 2880 and 2850 cm$^{-1}$, respectively. These two bands are due to the C-H groups in the benzene ring of CAC. In addition, the spectrum of CAC has a pronounced absorption band at 1471 cm$^{-1}$ (band c due to the C-C groups in the benzene ring) and at 732 cm$^{-1}$ (band d due to the out-of-plane C-H groups).

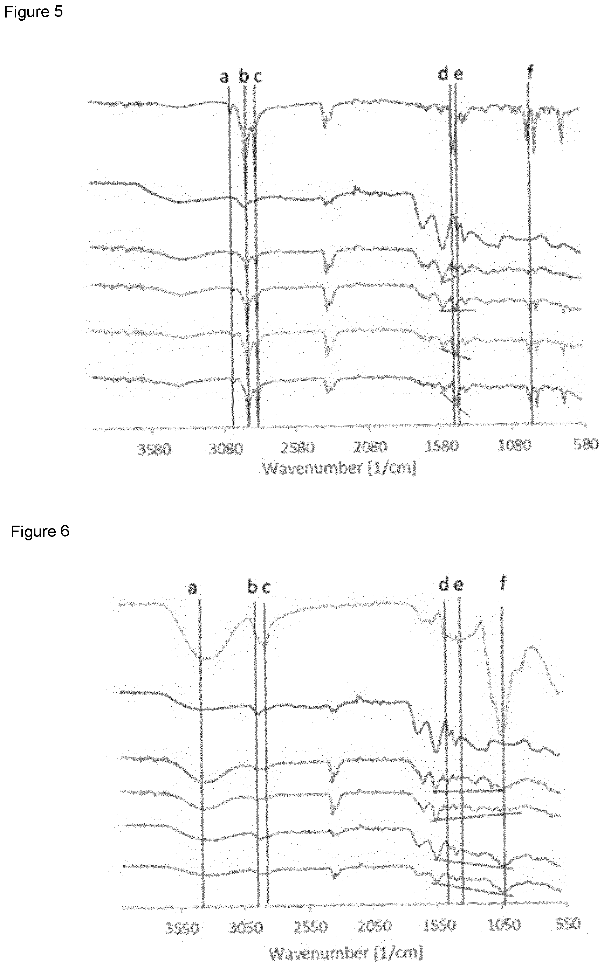

**Figure 5** shows FTIR spectra of samples 13 to 16 modified with TTABR and, for comparison, the spectra of TTABR and SAP alone. The spectra are shown as follows (from top to bottom): TTABR, SAP, sample 13, sample 14, sample 15, sample 16. Specific to TTABR are the bands at 2920 cm$^{-1}$ (b), 2850 cm$^{-1}$ (c) and 1471 cm$^{-1}$ (d), which are due to the long-chain alkyl residue of the quaternary ammonium compound. In addition, bands at 3016 cm$^{-1}$ (a), 1441 cm$^{-1}$ (e) and 950 cm$^{-1}$ (f) appear, which are caused by the cationic head group of the molecule.

Finally, **Figure 6** shows FTIR spectra of samples 17 to 20 modified with PQ10 compared to PQ10 and SAP alone. The spectra are shown as follows (from top to bottom): PQ10, SAP, sample 17, sample 18, sample 19, sample 20. The absorption spectrum of PQ10 has several characteristic bands, namely at 3312 cm$^{-1}$ (band a due to the hydroxyl groups), at 2925 cm$^{-1}$ and 2860 cm$^{-1}$ (bands b and c due to the saturated C-H groups), at 1434 cm$^{-1}$ and 1341 cm$^{-1}$ (bands d and e due to the $CH_2$ groups), and at 1065 cm$^{-1}$ (band f due to the C-O-C groups).

Overall, the prepared FTIR spectra confirmed that the produced particles contain SAP and the respective quaternary ammonium compound in different amounts as expected.

<u>3. Measurement of the absorption capacity</u>

The manufactured samples 1 to 20 and, for comparison, the unmodified SAP were examined with regard to their absorption capacity. For this purpose, a tea bag nonwoven measuring 125 x 85 mm (Degussa Evonik) was folded with the smooth side facing inwards and sealed on two sides with a welding device (Futura Junior 300 FJR from Audion Elektro). The sealing edges were each at least 5 mm wide. The bag was then filled with approximately 100 mg of sample material and the last open edge was sealed with the welding device. For the actual absorption test, the "tea bag" was immersed for 24 hours in a beaker filled with 500 ml demineralized water. The bag was then removed from the beaker, allowed to drain for 10 minutes in a hanging position and weighed. Each sample was analyzed three times in this way. The following formula was used to calculate the absorption capacity (FA):

$$FA \left[\frac{g}{g}\right] = \frac{P2 - P1 - BV}{P1}$$

In the above formula, FA, P1, P2 and BV have the following meanings:

FA: Absorption capacity of the analyzed sample material [g/g]
P1: Weight of the sample contained in the bag in dry condition [g]
P2: Weight of the bag filled with sample material after immersion in water for 24 hours and dripping for 10 minutes [g]
BV: Weight of an empty bag without sample material after immersion in water for 24 hours and dripping for 10 minutes [g]

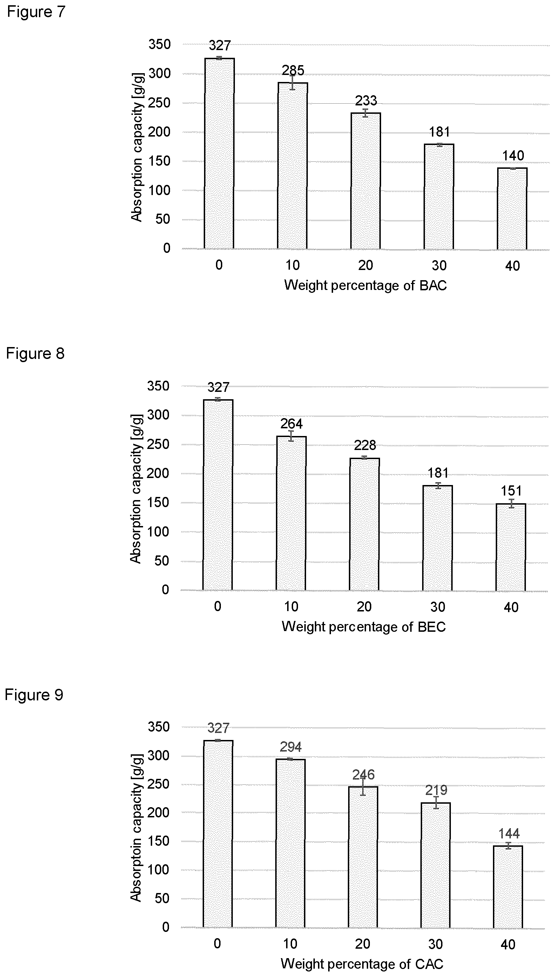

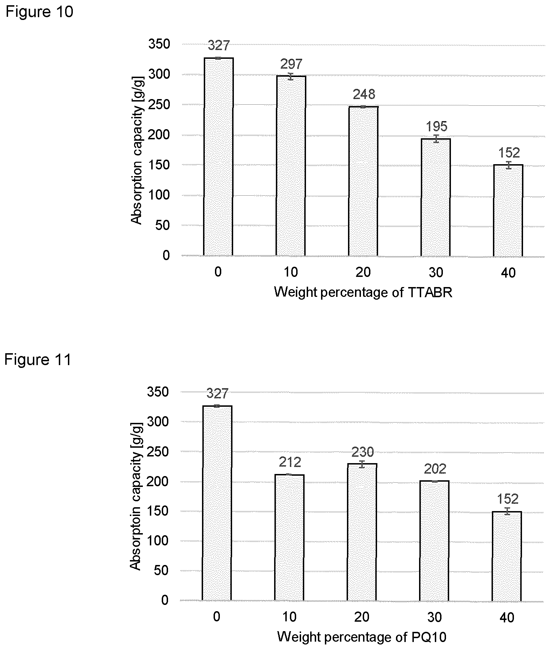

**Figures 7 to 11** show the absorption capacities determined. The absorption capacities shown are mean values from three individual determinations in each case. In each diagram, the absorption capacity of the unmodified SAP in the first bar is also shown for comparison, which was 327 $\pm$ 2 g/g. As can be seen from the diagrams, the absorption capacity of the modified SAP particles decreases almost linearly with increasing quaternary ammonium compound content. The different quaternary ammonium compounds behave relatively similarly. For example, the samples with the highest quat content of 40 % by weight only have an absorption capacity of around 150 g/g. Nevertheless, the absorption capacities of even the samples with a high quaternary ammonium compound content are still sufficient to absorb larger quantities of wound exudate and can therefore be used advantageously in wound treatment.

<u>4. Antimicrobial effect determined by agar diffusion test</u>

The agar diffusion test was used to evaluate the susceptibility of S. aureus (DSM 346) to selected examples of the prepared samples. An overnight bacterial culture ($10^9$ CFU/ml) was diluted to obtain a concentration of $10^7$ CFU/ml. 100 $\mu$l of the bacterial suspension was added to Muller-Hinton Agar (MHA) and a bacterial lawn was created by spreading the suspension with a sterile swab. The different modified SAP particles were moistened with Tryptic Soy Broth (TSB). 1 g of moistened SAP particles were placed in the centre of the MHA plate pre-inoculated with bacteria. For the negative control, 1 g of moistened non-modified SAP particles were used, and for positive control just bacteria. The plates were incubated at 37°C for 18 hours. After the incubation time, the zone of inhibition was measured and corrected to the diameter of the samples. The inhibition zone was calculated as follows:

$$H = \frac{D - d}{2}$$

In the above formula, H, D and d have the following meanings:

H: inhibition zone [mm]
D: total diameter [mm]
d: diameter of sample [mm]

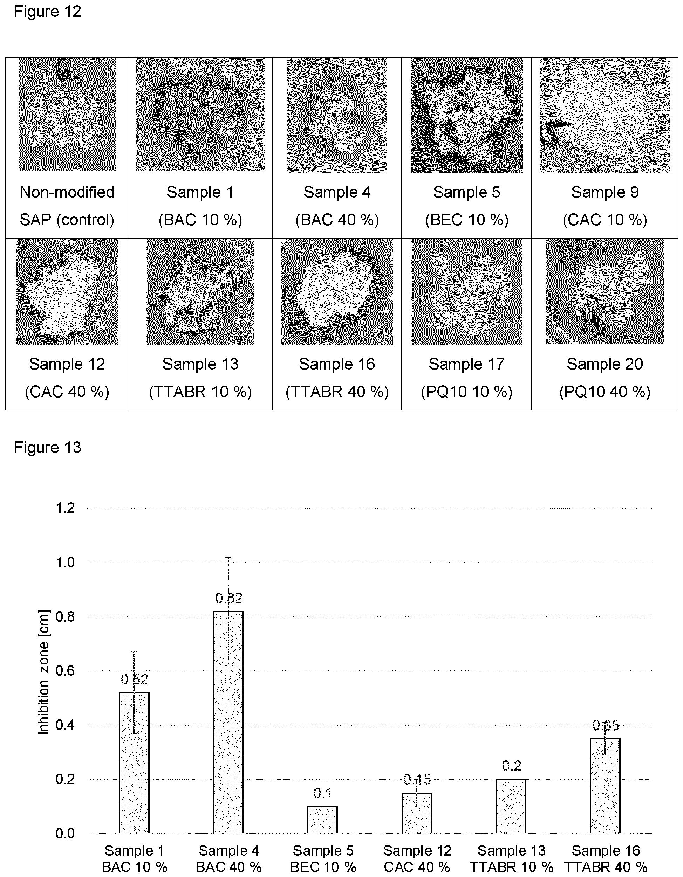

**Figure 12** shows the results of the agar diffusion tests for the test germ S. aureus. Except for the non-modified SAP (negative control) and the SAP samples modified with PQ10, the SAP samples tested showed antimicrobial activity against S. aureus at certain concentrations. The samples containing BAC, BEC and TTABR were already effective at the lower test concentration of 10 %, whereas the samples with CAC only showed an effect at the higher concentration of 40 %. The samples with the compound BAC and TTABR showed the best efficacy. The inhibition zones determined are shown in a bar diagram in **Figure 13.**

5. Antimicrobial effect according to AATCC TM100

The antimicrobial effect of the prepared samples was also determined in accordance with the AATCC TM100-2019 standard. A bacterial inoculum was prepared by growing a fresh 18 hour shake culture of S. aureus (DSM 346) and P. aeruginosa (DSM 1128) in sterile Tryptic Soy Broth (TSB, Sigma Aldrich (Poole, Dorset, UK)) at $37 \pm 2°C$ and 150 - 250 rpm. Thereafter, the overnight culture was diluted with fresh media to obtain a bacterial concentration between $1.0 \times 10^5$ and $3.0 \times 10^5$ CFU/ml. 1 g of moistened modified SAP particles, 1 g of moistened non-modified SAP particles as well as a control sample with the inoculum alone were used for the tests. As with the agar diffusion tests, the SAP-containing samples were pre-moistened with TSB before being tested. 1 ml of the bacteria inoculum was added to the different samples. Then, they were transferred to a flask. For the control sample, the inoculum was added to 100 ml of Muller Hinton Broth (MHB, Sigma Aldrich (Poole, Dorset, UK)). The recovery of bacteria immediately after inoculation (0 hours), after 4 hours and after 24 hours of incubation at 37°C was determined. After the designed incubation times, 100 ml of MHB was added to each jar, which were shaken vigorously. Then, the samples were sonicated for 5 minutes and serial dilutions in MHB were prepared and plated on agar plates. The plates were incubated for 24 hours at 37°C. Finally, the colony forming units (CFU) of each plate were reported. **Tables 2** and **3** contain the colony-forming units per ml (CFU/ml) counted for the samples tested at the start of the tests (immediate value), after 4 hours and finally after 24 hours. S. aureus (DSM 346) and in addition P. aeruginosa (DSM 1128) were used as test bacteria as already mentioned.

**Table 2:** Count values for the colony-forming units per ml (CFU/ml) for the test germ S. aureus

| sample No. | immediate value | 4 h value | 24 h value |
|---|---|---|---|
| control | $3.28 * 10^4$ | $2.98 * 10^5$ | $5.98 * 10^5$ |
| SAP | $3.28 * 10^4$ | $2.00 * 10^4$ | $6.38 * 10^5$ |
| 1 - BAC 10 % | $3.28 * 10^4$ | $2.93 * 10$ | 1.50 |
| 4 - BAC 40 % | $3.28 * 10^4$ | 0.00 | $4.48 * 10$ |
| 5 - BEC 10 % | $3.28 * 10^4$ | 1.00 | $1.27 * 10^4$ |
| 8 - BEC 40 % | $3.28 * 10^4$ | $2.38 * 10$ | $4.95 * 10^2$ |
| 9 - CAC 10 % | $3.28 * 10^4$ | $3.10 * 10^2$ | $1.08 * 10^2$ |
| 12 - CAC 40 % | $3.28 * 10^4$ | 5.25 | $3.25 * 10$ |
| 13- TTABR 10 % | $3.28 * 10^4$ | $4.69 * 10^2$ | $1.48 * 10^3$ |
| 16 - TTABR 40 % | $3.28 * 10^4$ | $4.65 * 10$ | $7.58 * 10^3$ |
| 17 - PQ10 10 % | $3.28 * 10^4$ | $1.83 * 10^4$ | $1.00 * 10^5$ |
| 20 - PQ10 40 % | $3.28 * 10^4$ | $1.88 * 10^4$ | $7.45 * 10^5$ |

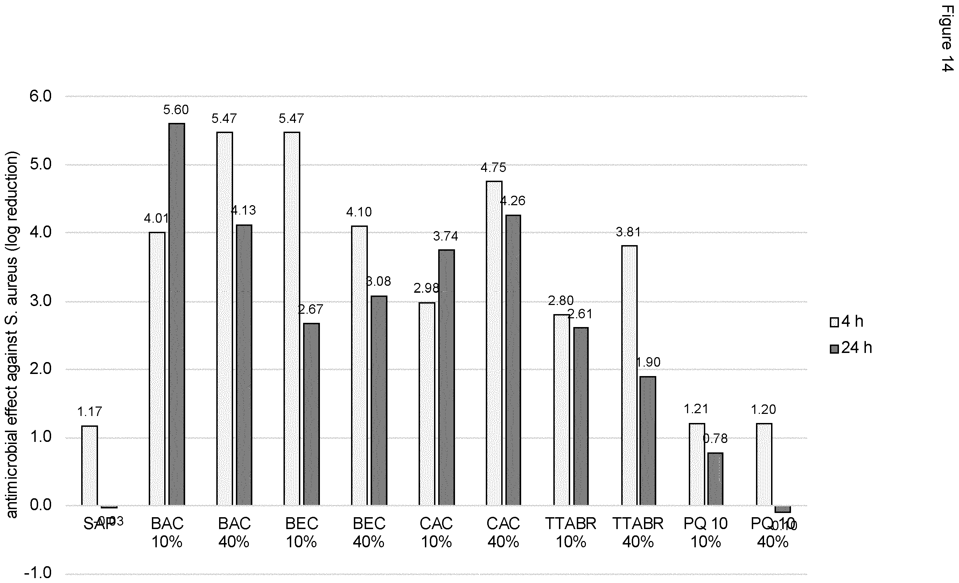

Note: a zero value was amended to 1 in order to calculate the log reduction shown in Figure 14

**Table 3:** Count values for the colony-forming units per ml (CFU/ml) for the test germ P. aeruginosa

| sample No. | immediate value | 4 h value | 24 h value |
|---|---|---|---|
| control | $3.38 * 10^4$ | $1.03 * 10^5$ | $4.80 * 10^6$ |

(continued)

| sample No. | immediate value | 4 h value | 24 h value |
|---|---|---|---|
| SAP | $3.38 * 10^4$ | $6.70 * 10^4$ | $1.17 * 10^5$ |
| 1 - BAC 10 % | $3.38 * 10^4$ | 7.00 | $5.10 * 10$ |
| 4 - BAC 40 % | $3.38 * 10^4$ | 0.00 | $1.35 * 10$ |
| 5 - BEC 10 % | $3.38 * 10^4$ | $1.28 * 10^3$ | $8.33 * 10^3$ |
| 8 - BEC 40 % | $3.38 * 10^4$ | $2.15 * 10^2$ | $3.00 * 10^4$ |
| 13 - TTABR 10 % | $3.38 * 10^4$ | $3.63 * 10^2$ | $5.30 * 10^3$ |
| 16 - TTABR 40 % | $3.38 * 10^4$ | $1.08 * 10$ | $1.70 * 10$ |
| 17- PQ10 10 % | $3.38 * 10^4$ | $7.90 * 10^4$ | $7.83 * 10^4$ |
| 20 - PQ10 40 % | $3.38 * 10^4$ | $5.13 * 10^4$ | $1.27 * 10^5$ |

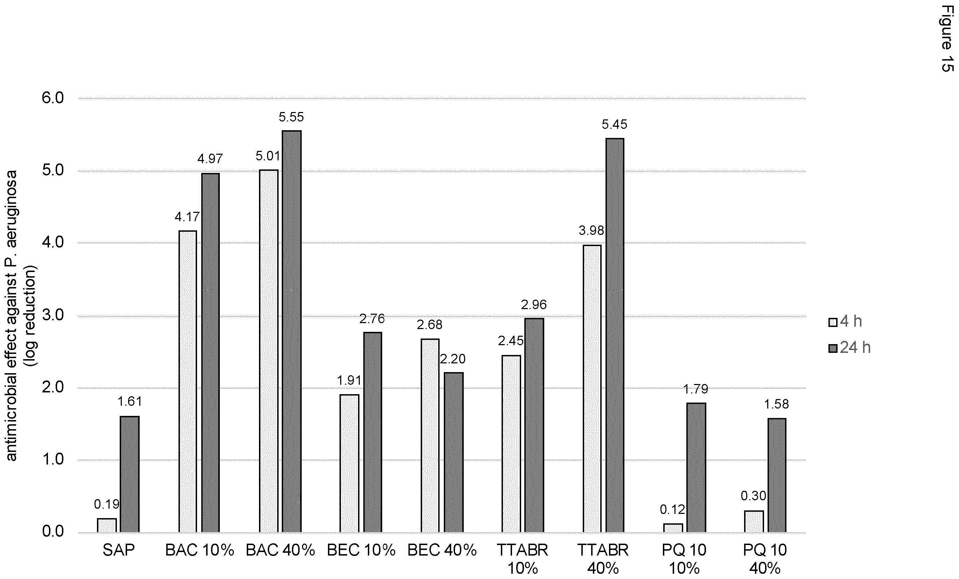

Note: a zero value was amended to 1 in order to calculate the log reduction shown in Figure 15

**Figures 14** and **15** show the results after 4 and 24 hours in the usual logarithmic form (log10 reduction in relation to the control) as bar diagrams. With the exception of the samples with PQ10, the modified SAP samples showed good efficacy against S. aureus (Figure 14). In particular, the samples with BAC were highly effective and showed a reduction factor of at least 4 log levels even at the low test concentration of 10 %. Selected samples were also tested against P. aeruginosa (figure 15). Here, too, good efficacy was shown at least for the SAP samples modified with TTABR and especially with BAC.

6. LPS and LTA binding studies as an indicator of an anti-inflammatory effect

Binding of LPS and LTA can be an indicator of anti-inflammatory efficacy. Accordingly, the samples produced were also examined using fluorescence microscopy and fluorescence spectroscopy to determine whether they can bind and thus neutralise LPS and LTA.

Preparation of fluorescence-labelled LPS

LPS was labelled with the fluorescent dye fluorescein isothiocyanate isomer I (FITC) according to the method of Skelly et al. (R. R. Skelly, P. Munkenbeck and D. C. Morrison, Infection and Immunity 1979, 23, 287 - 293). The fluorescently labelled LPS was prepared by incubating 10 mg R595 LPS and 40 mg FITC in 5.0 ml sodium borate (20 mM, pH 10.5) at 37°C for 3 hours. The FITC-labelled LPS was then purified by gel filtration (PD10 gel filtration columns) and stored protected from light at minus 20°C until use. LPS as well as FITC were purchased from Sigma-Adrich (Taufkirchen, Germany).

Preparation of fluorescence-labelled LTA

A reaction buffer, pH 8.3, was prepared by mixing PBS and 200 mM NaHCOs, pH 9, in a ratio of 20:1. Using the primary amino group of LTA from *Staphylococcus aureus,* a 1:1 ratio was calculated for the modification of LTA and the NHS conjugate of the fluorescent dye ATTO488 (Atto-Tec GmbH, Siegen, Germany). Accordingly, 200 $\mu$M) LTA and 200 $\mu$M) NHS fluorescent dye conjugate were incubated in the reaction buffer for 2 hours at 25°C. Subsequently, the labeled LTA was purified by gel filtration (PD10 gel filtration columns) and stored protected from light at minus 20°C until use.

Confocal laser scanning microscopy (CLSM)

For the experiments, 100 mg each of unmodified SAP (negative control) and modified SAP were pre-swollen in 30 ml PBS. The swollen SAP particles were then washed 5 times with 30 ml PBS. Following the washing step, the SAP particles were mixed with 2 ml 95 $\mu$M FITC-LPS or 2 ml 140 $\mu$M ATTO488-LTA in PBS and incubated for 24 hours at room temperature protected from light. The dye solution was then removed and the samples were washed again 5 times with 30 ml PBS. For the microscopic analyses, the SAP particles were mixed with 1 ml of a 1:1 mixture of glycerol and PBS (v/v). 20 $\mu$l of the suspension was applied to a microscope slide, covered with a coverslip and the edges sealed with Fixogum (Marabu GmbH, Tamm, Germany). Finally, CLSM analysis was performed using a Leica TCS SP8 system (Leica Microsystems, Wetzlar, Germany).

Quantitative fluorescence measurements

150 $\mu$l of sample or standard was used for photometric analysis using a SPARK Multimode Microplate Reader (Tecan Group, Männedorf, Switzerland). Measurements were performed in triplicate. Successive 1:2 dilutions with PBS were performed to generate the calibration curve, starting from a 5 $\mu$M FITC-LPS (ATTO488-LTA) stock solution up to 0.3125 $\mu$M FITC-LPS (ATTO488-LTA). Immediately before starting the measurement, the SAP samples were pipetted into the wells and the plate was shaken in the device.

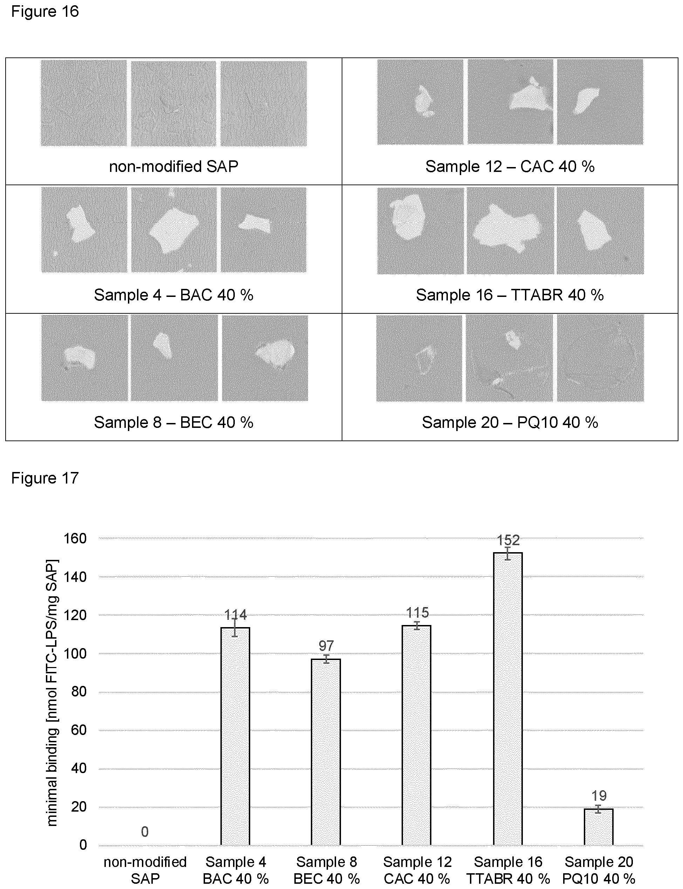

**Figure 16** shows images of the CLSM analyses performed regarding LPS binding. The best binding of FITC-LPS was found for the SAP particles modified with TTABR, followed by the SAP particles modified with BAC and with CAC. In the case of the SAP particles with BEC, slightly lower LPS binding was found compared to the aforementioned samples. Of the modified SAP samples analysed, the sample with PQ10 showed the lowest LPS binding. Quantitative fluorescence measurements with the microplate reader confirmed this trend (see **Figure 17**). In addition, the CLSM analyses showed FITC-LPS binding in the entire particle volume for most of the modified SAP particles. Only the sample modified with PQ10 showed FITC-LPS binding only in the periphery of the particles. This is because PQ10 cannot penetrate the SAP particles due to its high molecular weight, but merely envelops them (core-shell particles).

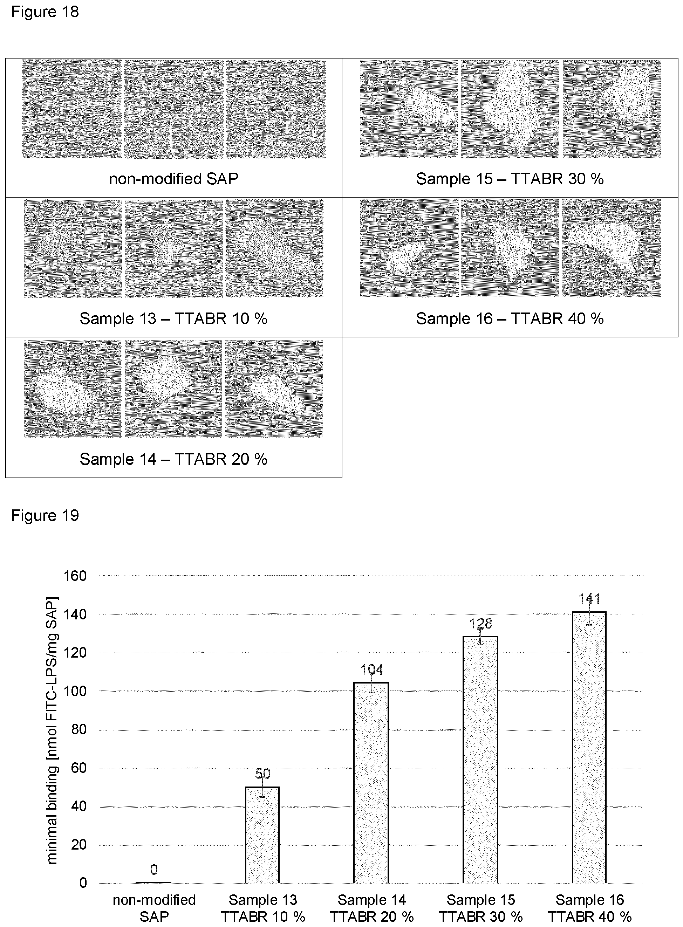

Since the best binding was found with SAP treated with the quaternary ammonium compound TTABR, the inventors further investigated FITC-LPS binding to SAP particles modified with different amounts of TTABR. As can be seen in **Figure 18,** comparatively weak binding was observed with the SAP sample containing 10 % TTABR. Much stronger binding was observed for the SAP sample with 20 % TTABR, which increased even further at 30 % and 40 % TTABR. This trend was confirmed by quantitative fluorescence measurements (**Figure 19**). These results indicate that the SAP particles should favourably comprise 20 % or more of the quaternary ammonium compound for optimal LPS binding.

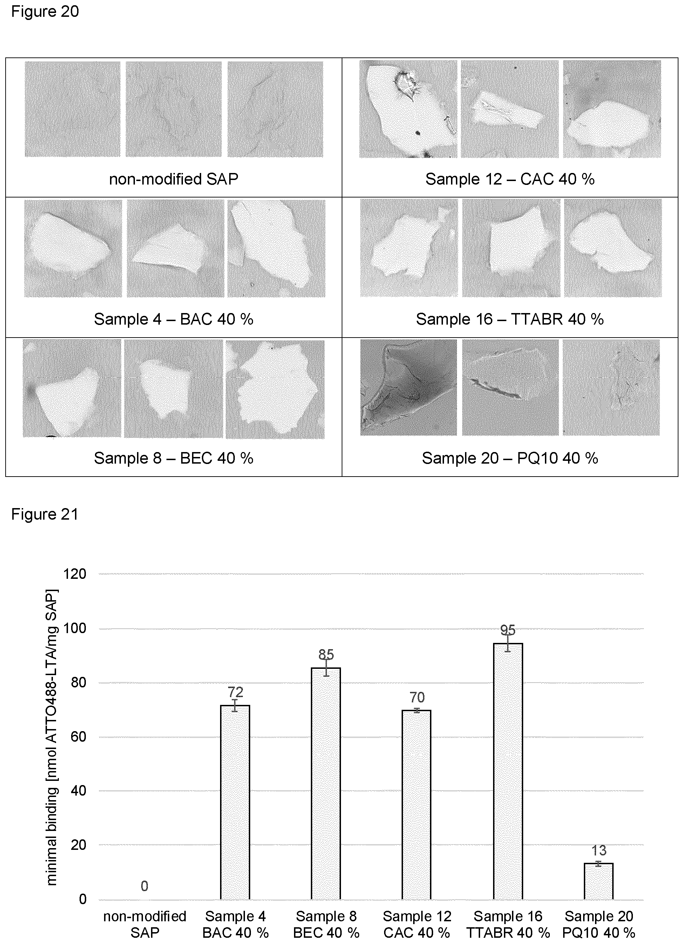

**Figure 20** shows images of the CLSM analyses performed for the LTA binding study. CLSM analysis showed that ATTO488-LTA binds best to SAP/TTABR particles, followed by binding to SAP/BEC particles. Slightly lower binding was observed for SAP/BAC particles and SAP/CAC particles. The weakest binding was observed for SAP/PQ10 particles. Quantitative fluorescence measurements with the microplate reader confirmed these results (**Figure 21**). In addition, the CLSM analyses for SAP modified with BAC, BEC, CAC and TTABR showed ATTO488-LTA binding over the entire particle volume. In contrast, ATTO488-LTA binding was only detectable in the periphery of the particles in the case of SAP particles modified with PQ10. Thus, the results from the LTA binding study are essentially consistent with the results from the LPS binding study described above.

The images in Figure 16, 18 and 20 have been converted to grey scale. Originally, the SAP particles with LPS and LTA binding activity appeared bright green due to the fluorescent dyes used.

7. MMP-2 and MMP-9 binding studies

In addition to LPS and LTA, the binding of MMP-2 and MMP-9 to BAC-modified SAP particles was also investigated using confocal laser scanning microscopy (CLSM).

Preparation of fluorescence-labelled MMP-2 and MMP-9

A reaction buffer, pH 8.3, was prepared by mixing PBS and 200 mM NaHCOs, pH 9, in a ratio of 20:1. To enable detection by fluorescence microscopy on the one hand, and to minimize the influence on the protein properties on the other, a ratio of 1:1 was calculated for the modification of the protein target (MMP-2, MMP-9) and the NHS conjugate of the fluorescent dye (ATTO643, Atto-Tec GmbH, Siegen, Germany). 14 $\mu$M protein and 14 $\mu$M NHS-fluorescent dye conjugate in reaction buffer were incubated for 2 hours at 25°C.

Subsequently, the labeled proteins were separated from the free dye by passing them over a NAP10 gel filtration column while buffered against PBS (thus achieving a final concentration of 9 $\mu$M ATTO643-MMP2/9), aliquoted, frozen in liquid nitrogen and stored at minus 20°C protected from light.

Confocal laser scanning microscopy (CLSM)

100 mg SAP/BAC (40 %) was allowed to swell for 24 hours in 50 ml PBS at 25°C. After that, the particles were washed five times with 30 ml PBS each time. Then 2 ml of 1 $_N$M ATTO643-MMP-2 or ATTO643-MMP-9 in PBS were added to the particles and incubated for 15 hours at 25°C protected from light. After five washes with 30 ml PBS each, the particles were analyzed by confocal laser scanning microscopy as described above.

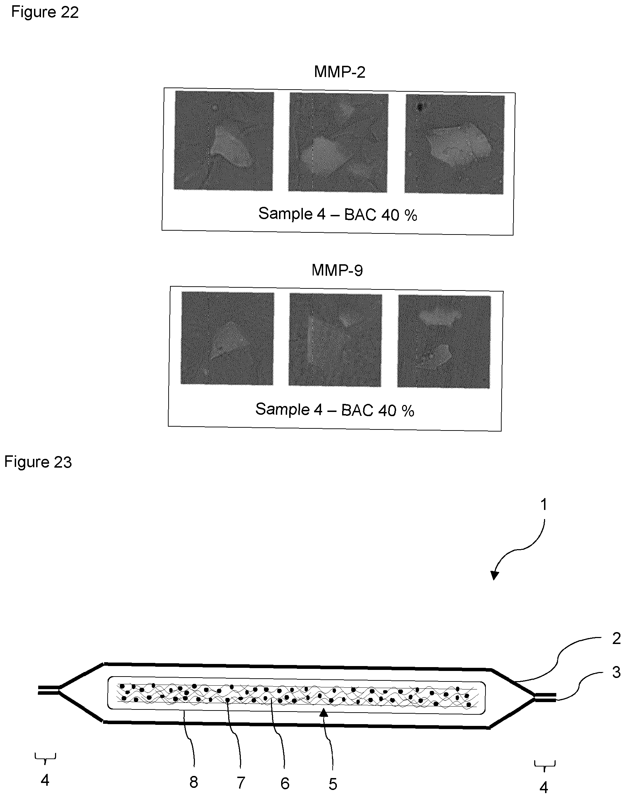

The results of the experiments are shown in **Figure 22.** It was found that the BAC-modified SAP particles can bind both MMP-2 and MMP-9. Again, the images in Figure 22 have been converted to grey scale. Originally, the SAP particles appeared bright red due to the fluorescent dye used.

**Wound dressing with the SAP particles according to the invention**

**Figure 23** shows a preferred embodiment of a wound dressing **1** with the SAP particles according to the invention in a lateral sectional view. The wound dressing **1** comprises a sheath. In the case shown, this comprises a first layer **2** forming a backing layer of the wound dressing **1** and a second layer **3** forming a wound contact layer of the wound dressing **1.** Both the first and the second layer **2, 3** are advantageously made of a nonwoven. The first layer **2** is preferably hydrophobic and essentially impermeable to liquids so that no wound exudate can be released from the wound dressing **1** to the outside. The second layer **3,** on the other hand, is typically hydrophilic and liquid-permeable so that wound exudate can penetrate the wound dressing. For example, the backing **2** can consist of synthetic plastic fibers, in particular polypropylene fibers, and the wound contact layer **3** can consist of a mixture of cellulose-based fibers and synthetic plastic fibers, in particular cellulose fibers and polypropylene fibers. In an edge area **4,** the two sheath layers **2, 3** are preferably joined by means of an ultrasonic weld.

In addition, the wound dressing **1** comprises an absorbent core **5** inside the sheath **2, 3**. The absorbent core **5** preferably consists of fluff pulp (cellulose fibers) **6,** in which the SAP particles **7** according to the invention are evenly distributed. For better fluid distribution within the wound dressing **1,** the absorbent core **5** can also be covered by an additional distribution layer **8**. This distribution layer **8** can be formed by a thin cellulose nonwoven, for example.

Overall, the wound dressing **1** is thus designed in the manner of a non-adhesive wound pad. Secondary dressing materials such as an adhesive tape can be used to attach the wound dressing **1** to the wound site.

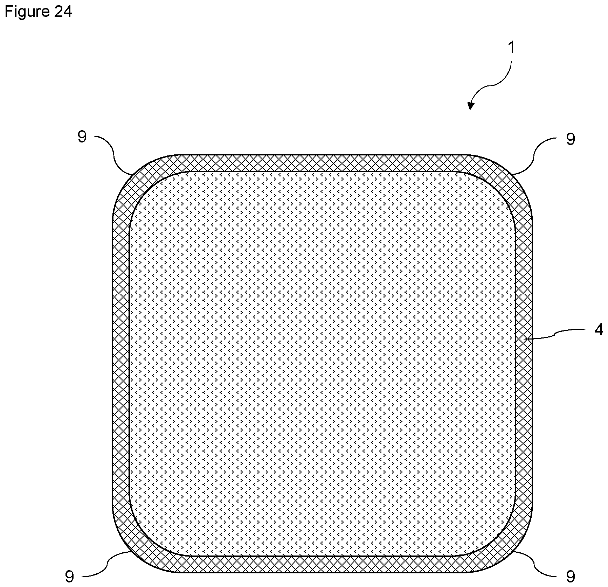

**Figure 24** shows the wound dressing **1** with a view of its upper side. Apart from the advantageously slightly rounded corners **9,** the wound dressing **1** is essentially rectangular in shape. Depending on the part of the body to be treated, however, the wound dressing **1** can also have a different shape, for example a round or oval shape.

**Claims**

1.  Particles comprising a superabsorbent polymer and a quaternary ammonium compound,

    wherein the superabsorbent polymer is a crosslinked polyacrylate and the quaternary ammonium compound is antimicrobial and/or anti-inflammatory, and
    wherein the quaternary ammonium compound is non-covalently linked to the superabsorbent polymer.

2.  Particles according to claim 1, wherein the particles consist essentially of the superabsorbent polymer and the quaternary ammonium compound.

3.  Particles according to claim 1 or 2, wherein the quaternary ammonium compound is selected from the group consisting of benzalkonium chloride, benzethonium chloride, cetalkonium chloride, tetradecyltrimethylammonium bromide and polyquaternium 10.

4.  Particles according to any one of the preceding claims, wherein the quaternary ammonium compound is linked to the superabsorbent polymer via ionic interactions.

5.  Particles according to any one of the preceding claims, wherein a positively charged functional group of the quaternary ammonium compound, in particular a quaternary nitrogen group, is linked to a negatively charged functional group of the superabsorbent polymer, in particular a carboxyl group, via ionic interactions.

6.  Particles according to any one of the preceding claims, wherein the particles comprise

    - 60 to 97.5 % by weight SAP, based on the total weight of the particles,
    - 2.5 to 40 % by weight of the quaternary ammonium compound, based on the total weight of the particles, and
    - optionally up to 6 % by weight water, based on the total weight of the particles.

7.  Particles according to any one of the preceding claims, wherein the particles have an absorption capacity of at least 100 g/g.

8.  Particles according to any one of the preceding claims, wherein the particles exhibit a log10 reduction of at least 2, preferably of at least 3 and in particular of at least 4 against S. aureus and/or P. aeruginosa over a test period of 24 hours.

9. Particles according to any one of the preceding claims, wherein the particles each comprise a core and a shell, wherein the superabsorbent polymer is contained in the core and the quaternary ammonium compound is contained in the shell.

10. Particles according to claim 9, wherein the quaternary ammonium compound is additionally contained in the core.

11. Process for producing modified particles, in particular particles according to any one of the preceding claims, comprising the following steps:

    i. providing superabsorbent polymer particles in the form of a crosslinked polyacrylate,
    ii. providing a quaternary ammonium compound,
    iii. dissolving the quaternary ammonium compound in water to form an aqueous solution of the quaternary ammonium compound,
    iv. dispersing the superabsorbent polymer particles in the solution to form a hydrogel,
    v. drying the hydrogel to obtain a substantially anhydrous modified polymer,
    vi. optionally comminuting and/or sieving the modified polymer to obtain a desired particle size of the modified polymer.

12. Particles obtainable by a process according to claim 11.

13. Particles according to any one of the claims 1 to 10 or 12 for use in medicine, in particular in wound treatment, in particular in wound treatment for binding LPS, LTA, MMP-2 and/or MMP-9.

14. Wound dressing comprising particles according to any one of the claims 1 to 10 or 12.

15. Wound dressing according to claim 14, wherein the wound dressing comprises a backing layer and an absorbent layer comprising the particles.

16. Wound dressing according to claim 15, wherein the absorbent layer forms an absorbent core of the wound dressing which is surrounded by a sheath.

17. Wound dressing according to any one of the claims 14 to 16, wherein the wound dressing further comprises particles consisting essentially of crosslinked polyacrylate, in particular crosslinked sodium polyacrylate.

Figure 1

Figure 2

Figure 3

Wavenumber [1/cm]

Figure 4

Wavenumber [1/cm]

Figure 5

Figure 6

Figure 7

Figure 8

Figure 9

Figure 10

Figure 11

Figure 12

| | | | | |
|---|---|---|---|---|
| Non-modified SAP (control) | Sample 1 (BAC 10 %) | Sample 4 (BAC 40 %) | Sample 5 (BEC 10 %) | Sample 9 (CAC 10 %) |
| Sample 12 (CAC 40 %) | Sample 13 (TTABR 10 %) | Sample 16 (TTABR 40 %) | Sample 17 (PQ10 10 %) | Sample 20 (PQ10 40 %) |

Figure 13

Figure 14

Figure 15

Figure 16

| non-modified SAP | Sample 12 – CAC 40 % |
| Sample 4 – BAC 40 % | Sample 16 – TTABR 40 % |
| Sample 8 – BEC 40 % | Sample 20 – PQ10 40 % |

Figure 17

Figure 18

| non-modified SAP | Sample 15 – TTABR 30 % |
| Sample 13 – TTABR 10 % | Sample 16 – TTABR 40 % |
| Sample 14 – TTABR 20 % | |

Figure 19

Figure 20

| non-modified SAP | Sample 12 – CAC 40 % |
|---|---|
| Sample 4 – BAC 40 % | Sample 16 – TTABR 40 % |
| Sample 8 – BEC 40 % | Sample 20 – PQ10 40 % |

Figure 21

Figure 22

MMP-2

Sample 4 – BAC 40 %

MMP-9

Sample 4 – BAC 40 %

Figure 23

Figure 24

Europäisches
Patentamt

European
Patent Office

Office européen
des brevets

# EUROPEAN SEARCH REPORT

**Application Number**

EP 24 21 3143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/010461 A1 (HERFERT NORBERT [DE] ET AL) 14 January 2010 (2010-01-14) * claims 1, 7, 9, 10, 11, 16, 18, 21, 22 paragraphs [0040], [0044], [0060-0061], [0065-0067], [0076-0078] * ----- | 1-9, 11-17 | INV. A61L15/24 A61L15/46 A61L15/60 |
| A | US 2024/374780 A1 (HAYNES JENNIFER [GB] ET AL) 14 November 2024 (2024-11-14) * paragraphs [0004-0007] Table 1 * ----- | 9 | |
| A | XIAOHONG LI ET AL: "Synthesis and water absorbency of polyampholytic hydrogels with antibacterial activity", JOURNAL OF APPLIED POLYMER SCIENCE, vol. 112, no. 1, 5 April 2009 (2009-04-05) , pages 439-446, XP055592598, US ISSN: 0021-8995, DOI: 10.1002/app.29409 * abstract page 2 - column 2 figures 6, 7 * ----- | 10 | |

| | TECHNICAL FIELDS SEARCHED (IPC) |
|---|---|
| | A61L |

The present search report has been drawn up for all claims

| Place of search | Date of completion of the search | Examiner |
|---|---|---|
| The Hague | 3 April 2025 | Lopez Serrano, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 3143

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2025

| Patent document cited in search report | Publication date | Patent family member(s) | Publication date |
|---|---|---|---|
| US 2010010461 A1 | 14-01-2010 | CN 101511917 A | 19-08-2009 |
| | | EP 2059551 A2 | 20-05-2009 |
| | | JP 2010501697 A | 21-01-2010 |
| | | US 2010010461 A1 | 14-01-2010 |
| | | WO 2008025655 A2 | 06-03-2008 |
| US 2024374780 A1 | 14-11-2024 | AU 2022279654 A1 | 18-01-2024 |
| | | BR 112023024800 A2 | 15-02-2024 |
| | | CA 3220424 A1 | 01-12-2022 |
| | | CN 118234378 A | 21-06-2024 |
| | | EP 4346408 A1 | 10-04-2024 |
| | | GB 2608270 A | 28-12-2022 |
| | | JP 2024522286 A | 13-06-2024 |
| | | KR 20240015103 A | 02-02-2024 |
| | | US 2024374780 A1 | 14-11-2024 |
| | | WO 2022248890 A1 | 01-12-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 9003-04-7
- *CHEMICAL ABSTRACTS*, 63449-41-2
- *CHEMICAL ABSTRACTS*, 121-54-0
- *CHEMICAL ABSTRACTS*, 122-18-9
- *CHEMICAL ABSTRACTS*, 1119-97-7
- *CHEMICAL ABSTRACTS*, 68610-92-4
- **R. R. SKELLY ; P. MUNKENBECK ; D. C. MORRISON**. *Infection and Immunity*, 1979, vol. 23, 287-293